(19) Europäisches Patentamt
European Patent Office
Office européen des brevets

(11) Veröffentlichungsnummer: **0 350 690**
**A2**

(12) **EUROPÄISCHE PATENTANMELDUNG**

(21) Anmeldenummer: 89111538.8

(51) Int. Cl.⁴: **C07K 15/06** , **A61K 39/395**

(22) Anmeldetag: 24.06.89

(30) Priorität: 14.07.88 DE 3823804

(43) Veröffentlichungstag der Anmeldung:
17.01.90 Patentblatt 90/03

(84) Benannte Vertragsstaaten:
**AT BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Moeller, Achim, Dr.**
**Wilhelm-Busch-Strasse 51**
**D-6703 Limburgerhof(DE)**
Erfinder: **Emling, Franz, Dr.**
**Valentin-Bauer-Strasse 22**
**D-6700 Ludwigshafen(DE)**
Erfinder: **Kurfuerst, Manfred, Dr.**
**Anilinstrasse 71**
**D-6733 Hassoch(DE)**
Erfinder: **Meyer, Thomas, Dr.**
**Gruenerstrasse 14 a**
**D-6700 Ludwigshafen(DE)**

(54) Neutralisation der in vitro- und in vivo-toxischen Eigenschaften von TNF-alfa durch monoklonale Antikörper und die davon abgeleiteten Fragmente.

(57) Es werden Fragmente des monoklonalen Antikörpers MAK 195 beschrieben, die sich zur Bekämpfung von Krankheiten eignen.

EP 0 350 690 A2

## Neutralisation der in vitro und in vivo toxischen Eigenschaften von TNF-α durch monoklonale Antikörper und die davon abgeleiteten Fragmente

Die vorliegende Erfindung beschreibt die Verwendung von von neutralisierenden anti-TNF-α monoklonalen Antikörpern abgeleiteten F(ab')₂- und F(ab)-Fragmenten zur Neutralisation der in vitro und in vivo toxischen Eigenschaften von TNF-α.

Tumornekrosefaktor-α (TNF-α) wurde zuerst bei antitumoralen Studien im Serum von Mäusen gefunden, wobei diese zuerst mit Bazillus Calmette-Guerin und anschließend mit Endotoxin injiziert wurden. (Carswell, E.A. et al; Proc. Natl. Acad. Sci., USA (1975), 72: 3666-3670). Diese anfänglichen Untersuchungen am TNF-α lenkten die Aufmerksamkeit besonders auf die anscheinend selektive Anti-Tumor-Funktion dieses Lymphokins. So wurden nekrotische Effekte auf verschiedene transplantable Tumore der Maus und cytotoxische Aktivität gegen Tumorzellen in Kultur gefunden (Pick, E. Lymphokines (1987), 14: 223-252).

Neuere Untersuchungen zeigten jedoch, daß TNF-α ein weites Spektrum an biologischen Aktivitäten auf verschiedene nicht maligne Zelltypen hat (Pick, E. Lymphokines (1987) 14: 203-223).

Besonders intensiv wurde die Rolle von TNF-α bei Studien zum Schock und bei Gewebeverletzungen untersucht. Ursprünglich wurde angenommen, daß dies durch einen direkten Effekt des LPS-Moleküls hervorgerufen wird. Untersuchungen von Cerami et al. in der Ratte zeigten jedoch, daß ein Sekretionsprodukt des Makrophagen, nämlich TNF-α, ein wichtiger Mediator des letalen Effektes von Endotoxin ist (Tracey, K.J. et al. Science (1986), 234: 470-474).

Beutler et al. konnte durch passive Immunisierung mit einem polyklonalen Antikörper gegen TNF-α zeigen, daß Mäuse resistent gegen den letalen Effekt von Endotoxin wurden (Science (1985) 229: 869-871). Diese Ergebnisse konnten mit monoklonalen Antikörpern in den Tiermodellen Maus und Pavian bestätigt werden (Shimamoto, Y. et al. Immun. Lett. (1988) 17: 311-318; Tracey, K.J. et al. Nature (1987), 330: 662-664).

Neben dieser zentralen Rolle beim septischen Schock zeigen neuere Untersuchungen das Vorhandensein von TNF-α bei Nierenabstoßungen, Transplantationen und Schocklunge (acute respiratory distress syndrom, ARDS) (Maury, C.P.J. and Teppo. A.-M., J. Exp. Med (1987) 166: 1132-1137). Piguet et al. zeigten, daß mit einem TNF-α-spezifischen polyklonalen Antikörper die Mortalität der Mäuse bei der GVHD (graft versus host disease, Transplantat-Wirt-Reaktion) stark reduziert werden konnte (J. Exp. Med. (1987) 166: 1280-1289).

Eine weitere Anwendung finden Antikörper bei der cerebralen Malaria (Grau, G.E., et al. Science (1987) 237: 1210-1212).

In der EP-OS 260 610 ist die Verwendung von monoklonalen Antikörpern zum Nachweis und zur Bestimmung von TNF-α, zur Bekämpfung von Krankheiten und zur Isolierung von TNF-α mittels Immunadsorptionschromatographie beschrieben.

Es wurde nun gefunden, daß auch die durch Spaltung der monoklonalen TNF-α-Antikörper mit Pepsin bzw. Papain erhaltenen Fragmente sehr gute Eigenschaften besitzen.

Die Fragmente F(ab')₂ und F(ab) wurden nach dem Fachman bekannten Methoden hergestellt und ihre Eigenschaften unter in vitro- und in vivo-Bedingungen getestet.

Die Neutralisation der zytotoxischen Wirkung von TNF-α auf L929-Zellen konnte durch die Fragmente erreicht werden. Unter in vivo-Bedingungen in der Maus wird eine letale Dosis von TNF-α durch die Pepsin- und Papain-Fragmente neutralisiert.

Die hohe Assoziationskonstante (größer $10^9$ l pro Mol) und Spezifität lassen diese Reagentien bei Krankheiten Verwendung finden, wo ein erhöhter TNF-α-Spiegel eine Rolle spielt. Dabei haben die Fragmente gegenüber den kompletten Antikörpern den Vorteil, daß sie leichter in das Gewebe eindringen und im Körper eine reduzierte Immunantwort in Bezug auf neue Antikörperbildung hervorruft.

Da die erfindungsgemäßen Fragmente TNF-α inaktivieren, können sie zur Behandlung von Krankheiten eingesetzt werden, bei denen die Konzentration an TNF-α im Blut erhöht ist, wie z.B. bei septischem Schock. Weiter kann bei folgenden Erkrankungen eine Behandlung mit den Fragmenten angezeigt sein: Transplantationen, GVHD, Allergien, Autoimmunkrankheiten, Erkrankungen des rheumatischen Formenkreises, Schocklungen, entzündliche Knochenerkrankungen, Blutgerinnungsstörungen und Verbrennungen.

Die folgenden Beispiele sollen die Erfindung näher erläutern.

Beispiel 1

In vitro-Neutralisation der zytotoxischen Aktivität von humanem TNF-α

Die zytotoxische Aktivität von TNF-α wurde, wie bei Aggarwal et al. (J. Biol. Chem. 1985, 260: 2345-2354) beschrieben, durch Lyse der Mauszell-Linie L929 (ATCC-Nr. CCL1) bestimmt. Bei Versuchen zur Neutralisation der zytotoxischen Aktivität von TNF-α durch das F(ab')₂- bzw. F(ab)-Fragment des monoklonalen Antikörpers mAK 195 wurde eine Konzentration an TNF gewählt, bei der mindestens 90 % der Zellen lysierten. Die Antikörper-Fragmente wurden in Medium auf eine Konzentration von 2 μg/ml eingestellt und in eins zu zwei Schritten in Mikrotiterplatten verdünnt. Zu jeder Antikörperfragmentlösung (0,1 ml) wurden 0,05 ml rekombinanter TNF (2 ng/ml) gegeben und 2 h bei Raumtemperatur inkubiert. Anschließend erfolgte Zugabe von 50 000 L929-Zellen in 0,05 ml Medium und nach einer Inkubation von 20-24 h im Brutschrank bei 37° C wurden die Zellen fixiert und mit Kristallviolett gefärbt.

Der schützende Effekt der Antikörperfragmente zeigte sich durch die Färbbarkeit von intakten Zellen. Die Fragmente hemmen die zytotoxische Aktivität von TNF.

Beispiel 2

In vivo Neutralisation von human TNF-α

Der schützende Effekt der F(ab')₂- und F(ab)-Fragmente gegen human TNF wurde unter in-vivo-Bedingungen in männlichen Balb/c-Mäusen untersucht. 4-6 Wochen alte Mäuse wurden randomisiert und in Gruppen von 5 bzw. 10 Tieren aufgeteilt. Die Substanzen wurden intravenös in die laterale Schwanzvene gegeben (Applikationsvolumen 10 ml/kg). 24 h vor der rhu TNF-α-Applikation wurden die Tiere mit LPS 0,5 mg/kg i.v. "geprimed". TNF-α und LPS wurden vor der Injektion in Puffer A (150 mM NaCl und 0, 18 % Rinderserumalbumin (Sigma, RIA-grade)), gelöst. Zur Messung der Neutralisierung wurde zuerst TNF gegeben, die mAK-Fragmente 15-30 min danach. Die Tötungsraten wurden nach 24 h bestimmt. In diesem Versuch zeigen die Fragmente eine starke Neutralisation von humanem TNF-α.

Beispiel 3

a) Herstellung von F(ab')₂-Fragmenten

Der monoklonale Antikörper mAK 195 wurde bei pH 4,5 in Gegenwart von 0,5M NaCl mit Pepsin gespalten (30 μg Pepsin/mg mAb; 90 min; 37° C). Ausfallende Fc-Fragmente wurden abzentrifugiert und der Überstand über eine Sephacryl® S-200 Gelfiltrationssäule (1,5 cm x 75 cm) chromatographiert. Die F(ab')₂-Fragmente enthaltenden Fraktionen wurden vereinigt und zur Entfernung von Antikörperresten über Protein-A Sepharose® filtriert.

b) Herstellung von Fab-Fragmenten

Der monoklonale Antikörper mAK 195 wurde bei pH 8,0 in Gegenwart von 0,5M NaCl mit Cystin-aktiviertem Papain (10 μg/mg mAb; 4 h; 37° C) gespalten. Anschließend wurde das Papain durch Zugabe von 20 mM Jodacetamid inaktiviert und das Gemisch über eine Sephacryl® S-200 Gelfiltrationssäule (1,5 cm x 75 cm) getrennt. Die Fab-Fragmente enthaltenden Fraktionen wurden vereinigt und zur Entfernung der Fc-Fragmente über Protein A Sepharose® filtriert.

Die Bezeichnungen "Sephacryl" und "Sepharose" sind Warenzeichen der Firma Pharmacia.

Ansprüche

1. F(ab')₂- und/oder F(ab')-Fragmente des monoklonalen Antikörpers mAK 195.

2. F(ab')₂- und/oder F(ab')-Fragmente des monoklonalen Antikörpers mAK 195 zur Verwendung bei der Bekämpfung von Krankheiten.